(19) Europäisches Patentamt
European Patent Office
Office européen des brevets

(11) Veröffentlichungsnummer: **0 180 833**
**B1**

(12) # EUROPÄISCHE PATENTSCHRIFT

(45) Veröffentlichungstag der Patentschrift: **19.07.89**

(51) Int. Cl.[4]: **C 07 D471/04**, A 61 K 31/505 // (C07D471/04, 239:00, 221:00)

(21) Anmeldenummer: **85113269.6**

(22) Anmeldetag: **18.10.85**

(54) **4-Oxo-pyrido[2,3]pyrimidin-Derivate, Verfahren zur deren Herstellung und diese ethaltende Arzneimittel.**

(30) Priorität: **19.10.84 DE 3438350**

(43) Veröffentlichungstag der Anmeldung: **14.05.86 Patentblatt 86/20**

(45) Bekanntmachung des Hinweises auf die Patenterteilung: **19.07.89 Patentblatt 89/29**

(84) Benannte Vertragsstaaten: **AT BE CH DE FR GB IT LI LU NL SE**

(56) Entgegenhaltungen:
**BE–A– 525 394**
**US–A– 3 673 184**
**ARZNEIMITTEL FORSCHUNG, Band 31 (II), Nr. 8, 1981, Seiten 1173-1177, Aulendorf, DE; H. MEYER et al.: "2-Aminodihydropyridine"**
**Die Akte enthält technische Angaben, die nach dem Eingang der Anmeldung eingereicht wurden und die nicht in dieser Patentschrift enthalten sind.**

(73) Patentinhaber: **GÖDECKE AKTIENGESELLSCHAFT**
**Salzufer 16**
**D-1000 Berlin 10 (DE)**

(72) Erfinder: **Kleinschroth, Jürgen, Dr.**
**Freiburger Strasse 13**
**D-7809 Denzlinge (DE)**
Erfinder: **Satzinger, Gerhard, Dr.**
**Im Mattenbühl 7**
**D-7809 Denzlingen (DE)**
Erfinder: **Mannhardt, Karl, Dr.**
**Pfauenstrasse 14**
**D-7807 Elzach-Oberprechtal (DE)**
Erfinder: **Hartenstein, Johannes, Dr.**
**Fohrenbühl 23**
**D-7801 Stegen-Wittental (DE)**
Erfinder: **Osswald, Hartmut, Prof. Dr.**
**Kiefernweg 1**
**D-7808 Waldkirch 2 (DE)**
Erfinder: **Weinheimer, Günter, Dr.**
**Sachsenstrasse 4**
**D-7809 Denzlingen (DE)**
Erfinder: **Fritschi, Edgar, Dr.**
**Am Scheuerwald 2**
**D-7811 St. Peter (DE)**

**EP 0 180 833 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 4-Oxo-pyrido [2,3-d] pyrimidin-Derivate der allgemeinen Formel I

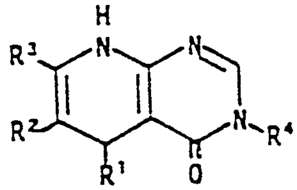

(I)

in welcher

$R^1$ einen unsubstituierten oder einen in 2- oder 3-Position durch Halogen-, Nitro-, Methyl- Methoxy-, Difluormethoxy-, Trifluormethoxy-, Dimethyl- oder Diethylamino Methylthio-, oder Trifluormethyl -substituierten oder einen vorzugsweise in 2,3- durch Methoxy- oder Methylendioxy- oder in 2,3- oder 2,6-Position durch Halogenatome, die gleich oder verschieden sein können, disubstituierten Phenylrest,

$R^2$ eine Nitrilgruppe, eine Carboxylgruppe oder einen Alkoxycarbonylrest der allgemeinen Formel II

$$—CO_2R^5 \qquad (II)$$

in welcher $R^5$ einen Methyl-, Ethyl-, n-Propyl-, Isopropyl-, n-Butyl-, Isobutyl-, tert. Butyl- oder einen Niederalkoxyalkylrest darstellt,

$R^3$ einen Mehtyl- oder Ethylrest oder eine Aminogruppe,

$R^4$ Wasserstoff, einen, Methyl-, Ethyl-, n-Propyl- oder Isopropylrest, eine Alkoxyalkylgruppe der allgemeinen Formel III

$$—(CH_2)_n—O—R^6 \qquad (III)$$

in welcher $R^6$ eine niedere geradkettige oder verzweigte Alkylgruppe darstellt und n den Wert 2 oder 3 bedeutet, oder eine Aminoalkylgruppe der allgemeinen Formel IV

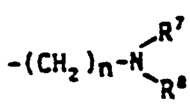

(IV)

in welcher $R^7$ und $R^8$ gleich oder verschieden sein können und eine geradkettige oder verzweigte niedere Alkylgruppe darstellen, oder gemeinsam eine niedere Alkylengruppe bilden und n gleich 2 oder 3 ist, bedeutet, sowie gegebenenfalls deren pharmakologisch unbedenkliche Salze.

Die Erfindung betrifft weiterhin ein Verfahren zur Herstellung von 4-Oxo-pyrido[2,3-d]pyrimidin-Derivaten der allgemeinen Formel VI

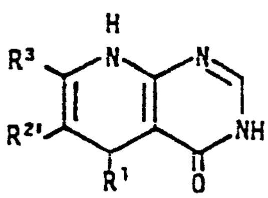

(VI)

in der $R^1$ und $R^3$ die oben angegebene Bedeutung haben und $R^{2'}$ eine Nitrilgruppe oder einen Alkoxycarbonylrest mit bis zu 6 Kohlenstoffatomen bedeutet, sowie ein Verfahren zur Herstellung von Verbindungen der allgemeinen Formel I, das dadurch gekennzeichnet ist, daß man Verbindungen der allgemeinen Formel VI in an sich bekannter Weise alkyliert, amino- oder alkoxyalkyliert.

Die Verbindungen der allgemeinen Formel VI werden hergestellt, indem man entweder

a) ein Dihydropyridin der allgemeinen Formel V

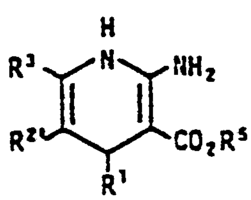

(V)

in welcher $R^1$, $R^{2'}$, $R^3$ und $R^5$ die oben angegebene Bedeutung haben, in Gegenwart einer Base mit s-Triazin umsetzt, oder

b) eine Verbindung der allgemeinen Formel VIII

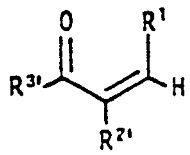

(VIII)

in welcher $R^1$ und $R^{2'}$ die oben angegebene Bedeutung haben und $R^{3'}$ eine geradkettige oder verzweigte Alkylgruppe mit bis zu 4 Kohlenstoffatomen bedeutet, durch Erhitzen in polaren Lösungsmitteln mit 6-Amino-4-hydroxypyrimidin kondensiert.

Die Herstellung der Verbindungen der allgemeinen Formel I erfolgt in an sich bekannter Weise durch Alkylierung bzw. Amino- oder Alkoxyalkylierung der Verbindungen der allgemeinen Formel VI mit Verbindungen der allgemeinen Formel VII

$$X—R^4 \qquad (VII)$$

in welcher $R^4$ die oben angegebene Bedeutung hat und X für Halogen, insbesondere Chlor, Brom oder Jod steht, vorzugsweise in Gegenwart eines Halogenwasserstoffakzeptors.

Verbindungen der allgemeinen Formel I, bei denen $R^2$ für eine Carboxylgruppe steht, werden hergestellt indem man Verbindungen der allgemeinen Formel I, in denen $R^2$ für einen zur Esterspaltung geeigneten Alkoxycarbonylrest steht, in an sich bekannter Weise vorzugsweise in saurem Medium hydrolysiert.

Die Verbindungen der allgemeinen Formel V (vgl. z. B. Liebig's Ann. Chem. 1977, S. 1895 ; Arzneim.-Forsch. 31 (II) 8 (1981) S. 1173) und VIII (vgl. z. B. Arch. Pharm. 317 (1984), S. 709) sind literaturbekannt oder können in analoger Weise hergestellt werden.

Zur Durchführung der Reaktion a) wird das Dihydropyridinderivat mit s-Triazin in einem inerten organischen Lösungsmittel in Gegenwart starker Basen wie z. B. Alkalialkoholaten oder Natriumhydrid in einem inerten organischen Lösungsmittel auf Temperaturen von 50-160 °C, vorzugsweise jedoch 100-150 °C, erhitzt. Als Solventien eignen sich hier vor allem polare Lösungsmittel wie Dimethylsulfoxid, Dimethylformamid oder Ethylenglykoldimethylether. Bei dieser Reaktion entstehen neben den jeweils als Hauptprodukt isolierten Verbindungen auch Verbindungen gemäß Patentanmeldung P 33 27 650, die durch Chromatographie abgetrennt werden.

Die Reaktion b) wird durch Erhitzen beider Komponenten in polaren Lösungsmitteln, vorzugsweise in Alkoholen bei Temperaturen von 60-120 °C, durchgeführt.

Die Alkylierung, Amino- und Alkoxyalkylierung der Verbindungen der allgemeinen Formel VI erfolgt nach allgemein bekannten Methoden, vorzugsweise unter Verwendung eines Halogenwasserstoffakzeptors. Die Reaktion verläuft bei der Wahl geeigneter Reaktionsbedingungen mit hoher Regioselektivität. Die zu erwartenden O-Alkylierungsprodukte werden überraschend nur in geringen Mengen gebildet. Die Trennung bzw. Reinigung der Produkte erfolgt mittels Chromatographie und/oder Kristallisation.

Saure oder basische Verbindungen der allgemeinen Formel I, welche für $R^2$ eine Carboxylgruppe bzw. für $R^4$ eine substituierte oder unsubstituierte Aminoalkylgruppe aufweisen, überführt man zum Zwecke der Reinigung und aus galenischen Gründen bevorzugt in kristalline, pharmakologisch verträgliche Salze.

Für den Fall, daß $R^2$ eine Carboxylgruppe darstellt, lassen sich mit Basen wie z. B. Hydroxiden oder Carbonaten entsprechende Salze der Alkali- oder Erdalkalimetalle herstellen. Wenn der Rest $R^3$ oder $R^4$ basischen Charakter aufweist, werden Salze in üblicher Weise durch Neutralisation der Basen mit entsprechenden anorganischen oder organischen Säuren erhalten. Als Säuren kommen z. B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Da die erfindungsgemäßen Verbindungen der allgemeinen Formel I an C-5 ein chirales Zentrum aufweisen, können sie entweder als racemische Gemische oder in Form der Enantiomeren vorliegen.

Bevorzugt sind Verbindungen der allgemeinen Formel I, in welcher

$R^1$ einen vorzugsweise in 2- oder 3- Position durch Halogen-, Nitro-, Methyl-, Methoxy-, Difluormethoxy-, Trifluormethoxy-, Dimethyl- oder Diethylamino-, Methylthio- oder Trifluormethyl- substituierten oder einen vorzugsweise in 2,3- Position durch Methoxy- oder Methylendioxy- oder in 2,3- oder 2,6- Position durch Halogenatome, die gleich oder verschieden sein können, disubstituierten Phenylrest,

$R^2$ eine Nitrilgruppe, eine Carboxylgruppe oder einen Alkoxycarbonylrest, insbesondere einen Methyl-, Ethyl-, Isopropyl-, Isobutyl- oder Methoxyethoxycarbonylrest,

$R^3$ einen Methyl- oder Ethylrest oder eine Aminogruppe,

$R^4$ Wasserstoff, einen Methyl-, Ethyl-, n-Propyl- oder Isopropylrest, eine Niederalkoxyalkylgruppe,

insbesondere die Ethoxyethylgruppe oder eine substituierte oder unsubstituierte Aminoalkylgruppe insbesondere die Dimethylaminopropyl- und die Piperidinopropylgruppe, bedeutet.

Die Verbindungen der allgemeinen Formel I weisen wertvolle pharmakologische Eigenschaften auf. Insbesondere zeigen sie als Calciumantagonisten gefäßspasmolytische, vasodilatatorische und antihypertensive Wirkungen. Einige Verbindungen zeigen überraschenderweise außerdem eine kontraktilitätserhöhende Wirkung.

Aufgrund ihrer gefäßspasmolytischen Wirkungen sind sie vor allem bei cerebralen, cardialen und peripheren Gefäßerkrankungen wie myokardialer Ischämie, bei cerebralem Infarkt, pulmonalen Thrombosen und bei Arteriosklerose und anderen stenotischen Erscheinungen indiziert.

Die 4-Oxo-pyrido [2,3-d] pyrimidin-Derivate der vorliegenden Erfindung sind daher wertvolle Mittel für die Bekämpfung der Herz-Kreislauf-Mortalität. Ein weiterer Gegenstand der vorliegenden Erfindung ist daher die Verwendung der 4-Oxo-pyrido [2,3-d] pyrimidine der allgemeinen Formel I bei der Bekämpfung von Gefäßerkrankungen.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z. B. Tartrat- und Citrat-Puffer, Ethanol, Komplexbildner (wie Äthylendiamintetraessigsäure und deren nichttoxische Salze) sowie hochmolekulare Polymere (wie flüssiges Polyäthylenoxid) zur Viskositätsregulierung. Feste Trägerstoffe sind z. B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten.

Die enteral verabreichten Einzeldosen liegen im Bereich von etwa 5 bis 250 mg, vorzugsweise 10-100 mg. Parenteral werden etwa 1 bis 20 mg gegeben.

Die folgenden Beispiele sollen die Erfindung näher erläutern :

## Beispiel 1

(±)-3,4,5,8-Tetrahydro-7-methyl-4-oxo-5-phenyl-pyrido[2,3-d]pyrimidin-6-carbonsäureethylester (Verfahren a)

Zu einer gerührten Suspension von 4,5 g (150 mMol) Natriumhydrid (80 %ig in Paraffinöl) in 75 ml trockenem Dimethylformamid wird unter Stickstoffatmosphäre eine Lösung von 40,6 g (123 mMol) (±)-2-Amino-1,4-dihydro-6-methyl-4-phenylpyridin-3,5-dicarbonsäurediethylester in 200 ml Dimethylformamid getropft. Bei Nachlassen der Gasentwicklung wird noch 30 Minuten bei Raumtemperatur gerührt; anschließend werden 10,0 g (123 mMol) s-Triazin in 250 ml Dimethylformamid zugetropft. Die Reaktionsmischung wird 16 Stunden auf 110 °C erhitzt und nach dem Abkühlen im Vakuum eingeengt. Der dunkle Rückstand wird an Kieselgel mit Dichlormethan/Methanol 95 : 5 chromatographiert. Die Fraktion mit dem $R_f$ 0,5 wird isoliert, mit Aceton zum Sieden erhitzt und die nach dem Abkühlen ausgefallenen Kristalle zur weiteren Reinigung aus Ethanol umkristallisiert.

Man erhält (±)-3,4,5,8-Tetrahydro-7-methyl-4-oxo-5-phenylpyrido [2,3-d] pyrimidin-6-carbonsäureethylester in Form beiger Kristalle vom Schmp. 303-305 °C (Z).

In analoger Weise werden die folgenden Verbindungen erhalten :

(±)-5-(2-Fluorphenyl)-3,4,5,8-tetrahydro-7-methyl-4-oxo-pyrido[2,3-d] pyrimidin-6-carbonsäureethylester (1.a), Verfahren a) Schmp. 264-265 °C aus Ethanol

(±)-3,4,5,8-Tetrahydro-7-methyl-5-(2-nitrophenyl)-4-oxo-pyrido-[2,3-d] pyrimidin-6-carbonsäureethylester (1.b), Verfahren a) Schmp. 280-282 °C aus Ethanol

(±)-3,4,5,8-Tetrahydro-7-methyl-4-oxo-5-(2-trifluormethylphenyl)-pyrido [2,3-d] pyrimidin-6-carbonsäuremethylester (1.c), Verfahren a) Schmp. 294-295 °C aus Essigsäureethylester/Ethanol

(±)-7-Amino-3,4,5,8-tetrahydro-5-(2-methoxyphenyl)-4-oxo-pyrido [2,3-d] pyrimidin-6-carbonsäureethylester (1.d), Verfahren a) Schmp. 283-285 °C aus Ethanol

(±)-3,4,5,8-Tetrahydro-7-methyl-5-(3-nitrophenyl)-4-oxo-pyrido [2,3-d] pyrimidin-6-carbonsäureisopropylester (1.e), Verfahren a) Schmp. 258-260 °C aus Isopropanol

## Beispiel 2

(±)-3,4,5,8-Tetrahydro-7-methyl-5-(3-nitrophenyl)-4-oxo-pyrido [2,3-d] pyrimidin-6-carbonsäuremethylester (Verfahren b)

17,5 g (70 mMol) 3-Nitrobenzylidenacetessigsäuremethylester und 7,8 g (70 mMol) 4-Amino-6-hydroxypyrimidin werden in 800 ml trockenem Ethanol 20 Stunden unter Rückfluß erhitzt. Die nach dem Abkühlen ausgefallenen Kristalle liefern nach Umkristallisation aus Ethanol farblose Nadeln vom Schmp. 289-290 °C.

Beispiel 3

(±)-5-(2-Fluorphenyl)-3,4,5,8-tetrahydro-3-isopropyl-7-methyl-4-oxo-pyrido [2,3-d] pyrimidin-6-carbonsäureethylester

Zu einer gerührten Suspension von 0,5 g (17 mMol) Natriumhydrid (80 %ig in Paraffinöl) in 30 ml trockenem Dimethylformamid wird eine Lösung von 3,7 g (11 mMol) (±)-5-(2-Fluorphenyl)-3,4,5,8-tetrahydro-7-methyl-4-oxo-pyrido [2,3-d] pyrimidin-6-carbonsäureethylester in 30 ml Dimethylformamid getropft. Bei Nachlassen der Gasentwicklung wird noch 30 Minuten bei Raumtemperatur gerührt; anschließend werden 2,6 g (15 mMol) Isopropyljodid in 15 ml Dimethylformamid zugetropft. Es wird 20 Stunden bei Raumtemperatur gerührt, das Lösungsmittel im Vakuum abrotiert und der Rückstand mit 100 ml Wasser verrührt.

Die gebildeten Kristalle werden abfiltiert, getrocknet, in Essigsäureethylester gelöst und an Kieselgel mit Toluol/Essigsäureethylester 1 : 1 chromatographiert.

Die Fraktion mit dem $R_f$ 0,3 wird isoliert und aus Diisopropylether/Ethanol umkristallisiert. Man erhält (±)-5-(2-Fluorphenyl)-3,4,5,8-tetrahydro-3-isopropyl-7-methyl-4-oxo-pyrido [2,3-d] pyrimidin-6-carbonsäureethylester in Form farbloser Kristalle vom Schmp. 178-180 °C.

In analoger Weise werden die folgenden Verbindungen erhalten :

(±)-3,4,5,8-Tetrahydro-3-isopropyl-7-methyl-4-oxo-5-phenyl-pyrido [2,3-d] pyrimidin-6-carbonsäureethylester (3.a) Schmp. 184-185 °C aus Diisopropylether/Ethanol

(±)-3,4,5,8-Tetrahydro-7-methyl-5-(3-nitrophenyl)-4-oxo-3(3-piperidinopropyl)-pyrido [2,3-d] pyrimidin-6-carbonsäureisopropylester (3.b) Schmp. 143-145 °C aus Diisopropylether

Die folgenden Vergleichsversuche veranschaulichen die pharmakologische Wirksamkeit der Verbindungen gemäß der allgemeinen Formel I :

a) Isolierte glatte Muskeln (Tabelle 1)

von Kaninchen (Gefäßringe, A. basilaris, A. coronaria, A. saphena) werden im Organbad so eingespannt, daß isometrische Kontraktionen gemessen werden können. Die Kontraktion wird durch eine Kaliumdepolarisation in Tyrodelösung ausgelöst. Diese Versuchsanordnung ist ein bekanntes Standardmodell zur Erkennung von Substanzen, die die in der Kaliumdepolarisation geöffneten Calciumkanäle blockieren (Fleckenstein, Calcium Antagonism in Heart & Smooth Muscle, J. Wiley & Sons, 1983).

b) Isolierte Papillarmuskel (Tabelle 2)

Papillarmuskel aus der linken Herzkammer des Meerschweinchens werden wie bei isolierten Gefäßen im Organbad zur isometrischen Kontraktionsmessung eingespannt und durch Feldreizung mit einer Frequenz von 250/min (Reizdauer 10 msec, Amplitude supramaximal) elektrisch gereizt.

c) Spontanhypertensive Ratte (Tabelle 3)

In diesem Versuchsmodell von genetisch bedingtem Hypertonus wurde Beispiel Nr. Ia auf antihypertensive Wirkung getestet. Wie aus Tabelle 3 ersichtlich, ist eine gute und dosisabhängige andauernde antihypertensive Wirkung nach oraler Verabreichung zu beobachten.

d) Narkotisierte Ratte

In diesem Versuchsmodell der Inaktin-narkotisierten Ratte wurde Beispiel Nr. Ia in einer Dosierung von 100 mg/kg i. d. getestet (n = 3). Die Herzrate verblieb unverändert, während der Blutdruck um 40 % unter den Ausgangswert fiel (75 Min p. a.).

(Siehe Tabelle 1 Seite 6 f.)

Tabelle 1

Konzentrationen ($IC_{50}$, mol/l) von Verbindungen, die eine halbmaximale Hemmung der $K^+$-Depolarisationskontraktur der Gefäßringe im Organbad bewirken. A. bas. = Arteria basilaris, A. cor. = Arteria coronaria, A. saph. = Arteria saphena des Kaninchens ; mittlerer Durchmesser 0,5-1,0 mm.

| Beisp.Nr. | A. basilaris | A. coronaria | A. saphena |
|---|---|---|---|
| 1 | $8 \times 10^{-6}$ | $1 \times 10^{-5}$ | $7 \times 10^{-6}$ |
| 1a | $1.8 \times 10^{-6}$ | $1.5 \times 10^{-6}$ | $1.2 \times 10^{-6}$ |
| 3 | $2 \times 10^{-5}$ | $2 \times 10^{-5}$ | $9 \times 10^{-6}$ |
| 1b | $\sim 4 \times 10^{-7}$ | $\sim 1 \times 10^{-6}$ | |
| 3a | $4 \times 10^{-8}$ | $4.5 \times 10^{-8}$ | |

Tabelle 2

Änderungen der Kontraktionsamplitude von isolierten Papillarmuskeln des Meerschweinchens (Reizfrequenz 250/min, Reizdauer 10 msec, Reizamplitude 10-20 V Feldreizung). IC = Hemmkonzentration, ED = effektive Konzentration in mol/l. Indices 50 und 100 entsprechen halbmaximaler und maximaler Wirkung. Δ % = prozentuale Änderung der Kontraktionsamplitude gegenüber der Kontrolle.

| Beisp.Nr. | $IC_{100}$ | Δ % | $IC_{50}$ | Δ % |
|---|---|---|---|---|
| 1 | $10^{-4}$ | - 27 | $2 \cdot 10^{-5}$ | - 13 |
| | $ED_{100}$ | Δ % | $ED_{50}$ | Δ % |
| 1a | $10^{-6}$ | + 52 | $1,5 \cdot 10^{-8}$ | + 26 |
| 3 | $10^{-6}$ | + 30 | $2,2 \cdot 10^{-8}$ | + 15 |

Tabelle 3

Wirkung von Beispiel Nr. Ia auf den systolischen Blutdruck von spontanhypertensiven Ratten (SHR)

| Beisp.Nr. | Dosis mg/kg oral | Änderung des systol. Blutdrucks in % Zeitpunkt nach Applikation | | | |
|---|---|---|---|---|---|
| | | 30 min. | 120 min. | 240 min. | 24h |
| 1a | 100 | - 40 | - 19 | + 2 | - 4 |
| | 50 | - 32 | - 8 | + 1 | + 4 |
| | 25 | - 24 | - 2 | - 1 | + 5 |
| | 12,5 | - 11 | - 4 | + 2 | - 1 |

**I. Patentansprüche** (für die Vertragsstaaten : BE, CH, DE, FR, GB, IT, LI, LU, NL, SE)

1. 4-Oxo-pyrido [2,3-d] pyrimidin-Derivate der allgemeinen Formel I

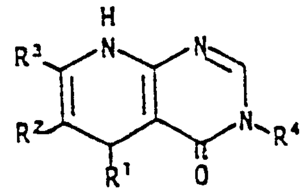

(I)

in welcher

$R^1$ einen unsubstituierten oder einen in 2- oder 3-Position durch Halogen-, Nitro-, Methyl-, Methoxy-, Difluormethoxy-, Trifluormethoxy-, Dimethyl- oder Diethylamino-, Methylthio-, oder Trifluormethyl-substituierten oder einen vorzugsweise in 2,3- durch Methoxy- oder Methylendioxy oder in 2,3- oder 2,6-Position durch Halogenatome, die gleich oder verschieden sein können, disubstituierten Phenylrest,

$R^2$ eine Nitrilgruppe, eine Carboxylgruppe oder einen Alkoxycarbonylrest der allgemeinen Formel II

$$—CO_2R^5 \qquad (II)$$

in welcher $R^5$ einen Methyl-, Ethyl-, n-Propyl-, Isopropyl-, n-Butyl-, Isobutyl-, tert. Butyl- oder einen Niederalkoxyalkylrest darstellt.

$R^3$ einen Methyl- oder Ethylrest oder eine Aminogruppe,

$R^4$ Wasserstoff, einen Methyl-, Ethyl-, n-Propyl- oder Isopropylrest, eine Alkoxyalkylgruppe der allgemeinen Formel III

$$—(CH_2)_n—O—R^6 \qquad (III)$$

in welcher $R^6$ eine niedere geradkettige oder verzweigte Alkylgruppe darstellt und n den Wert 2 oder 3 bedeutet, oder eine Aminoalkylgruppe der allgemeinen Formel IV

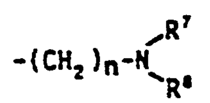

(IV)

in welcher $R^7$ und $R^8$ gleich oder verschieden sein können und eine geradkettige oder verzweigte niedere Alkylgruppe darstellen, oder gemeinsam eine niedere Alkylengruppe bilden und n gleich 2 oder 3 ist, bedeutet, sowie gegebenenfalls deren pharmakologisch unbedenkliche Salze.

2. Verfahren zur Herstellung von 4-Oxo-pyrido [2,3-d] pyrimidin-Derivaten gemäß Anspruch 1, dadurch gekennzeichnet, daß man entweder

a) ein Dihydropyridin der allgemeinen Formel V

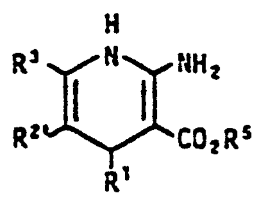

(V)

in welcher $R^1$, $R^3$ und $R^5$ die oben angegebene Bedeutung haben und R2′ eine Nitrilgruppe oder einen Alkoxycarbonylrest der allgemeinen Formel II bedeutet, mit s-Triazin kondensiert und die so erhaltenen Verbindungen der allgemeinen Formel VI

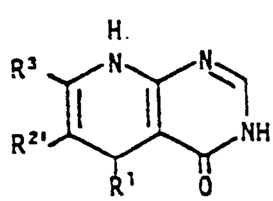

(VI)

in welcher $R^1$, $R^{2'}$ und $R^3$ die oben genannte Bedeutung haben, gegebenenfalls in an sich bekannter Weise mit einer Verbindung der allgemeinen Formel VII

$$X—R^4 \quad (VII)$$

in welcher $R^4$ mit Ausnahme von Wasserstoff die oben angegebene Bedeutung besitzt und X für Halogen, insbesondere Chlor, Brom oder Jod steht, umsetzt oder

b) eine Verbindung der allgemeinen Formel VIII

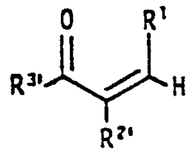

(VIII)

in welcher $R^1$ und $R^{2'}$ die oben angegebene Bedeutung haben und $R^{3'}$ eine geradkettige oder verzweigte Alkylgruppe mit bis zu 4 Kohlenstoffatomen bedeutet, mit 6-Amino-4-hydroxypyrimidin kondensiert und die so erhaltenen Verbindungen der allgemeinen Formel VI gewünschtenfalls mit einer Verbindung der allgemeinen Formel VII umsetzt.

3. Verwendung von Verbindungen der allgemeinen Formel I gemäß Anspruch 1, für die Herstellung von Arzneimitteln zur Bekämpfung von Gefäßerkrankungen.

4. Arzneimittel enthaltend mindestens eine Verbindung nach Anspruch 1.

**Patentansprüche** (für den Vertragsstaat AT)

1. Verfahren zur Herstellung von 4-Oxo-pyrido [2,3-d] pyrimidin-Derivaten der allgemeinen Formel I

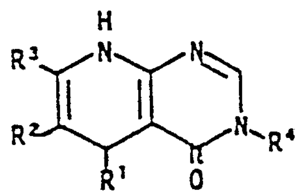

(I)

in welcher

$R^1$ einen unsubstituierten oder einen in 2- oder 3-Position durch Halogen-, Nitro-, Methyl-, Methoxy-, Difluormethoxy-, Trifluormethoxy-, Dimethyl- oder Diethylamino-, Methylthio-, oder Trifluormethyl-substituierten oder einen vorzugsweise in 2.3- durch Methoxy- oder Methylendioxy- oder in 2,3- oder 2,6-Position durch Halogenatome, die gleich oder verschieden sein können, disubstituierten Phenylrest,

$R^2$ eine Nitrilgruppe, eine Carboxylgruppe oder einen Alkoxycarbonylrest der allgemeinen Formel II

$$—CO_2R^5 \quad (II)$$

in welcher $R^5$ einen Methyl-, Ethyl-, n-Propyl-, Isopropyl-, n-Butyl-, Isobutyl-, tert. Butyl- oder einen Niederalkoxyalkylrest darstellt,

$R^3$ einen Methyl- oder Ethylrest oder eine Aminogruppe,

$R^4$ Wasserstoff, einen Methyl-, Ethyl-, n-Propyl- oder Isopropylrest, eine Alkoxyalkylgruppe der allgemeinen Formel III

$$—(CH_2)_n—O—R^6 \quad (III)$$

in welcher $R^6$ eine niedere geradkettige oder verzweigte Alkylgruppe darstellt und n den Wert 2 oder 3 bedeutet, oder eine Aminoalkylgruppe der allgemeinen Formel IV

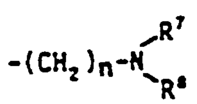

(IV)

in welcher $R^7$ und $R^8$ gleich oder verschieden sein können und eine geradkettige oder verzweigte niedere Alkylgruppe darstellen, oder gemeinsam eine niedere Alkylengruppe bilden und n gleich 2 oder 3 ist, bedeutet, sowie gegebenenfalls deren pharmakologisch unbedenkliche Salze, dadurch gekennzeichnet, daß man

a) ein Dihydropyridin der allgemeinen Formel V

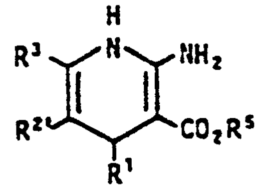

(V)

in welcher $R^1$, $R^3$ und $R^5$ die oben angegebene Bedeutung haben und $R^{2'}$ eine Nitrilgruppe oder einen Alkoxycarbonylrest der allgemeinen Formel II bedeutet, mit s-Triazin kondensiert und die so erhaltenen Verbindungen der allgemeinen Formel VI

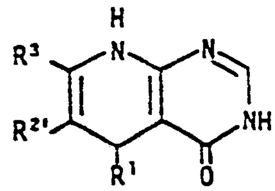

(VI)

in welcher $R^1$, $R^{2'}$ und $R^3$ die oben genannte Bedeutung haben, gegebenenfalls in an sich bekannter Weise mit einer Verbindung der allgemeinen Formel VII

$$X—R^4 \quad (VII)$$

in welcher $R^4$ mit Ausnahme von Wasserstoff die oben angegebene Bedeutung besitzt und X für Halogen, insbesondere Chlor, Brom oder Jod steht, umsetzt oder

b) eine Verbindung der allgemeinen Formel VIII

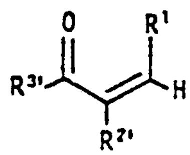

(VIII)

in welcher $R^1$ und $R^{2'}$ die oben angegebene Bedeutung haben und $R^{3'}$ eine geradkettige oder verzweigte Alkylgruppe mit bis zu 4 Kohlenstoffatomen bedeutet, mit 6-Amino-4-hydroxypyrimidin kondensiert und die so erhaltenen Verbindungen der allgemeinen Formel VI gewünschtenfalls mit einer Verbindung der allgemeinen Formel VII umsetzt und die so erhaltenen Verbindungen I anschließend gegebenenfalls in deren pharmakologisch verträglichen Salze überführt.

2. Verwendung von Verbindungen der allgemeinen Formel I gemäß Anspruch 1, für die Herstellung von Arzneimitteln zur Bekämpfung von Gefäßerkrankungen.

**Claims** (for the Contracting States : BE, CH, DE, FR, GB, IT, LI, LU, NL, SE)

1. 4-Oxo-pyrido [2,3-d] pyrimidine derivatives of the general formula I

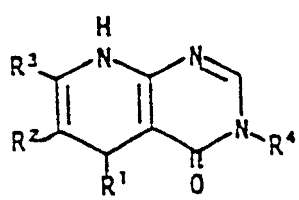

(I)

in which

$R^1$ represents an unsubstituted phenyl residue or a substituted phenyl residue, substituted in the 2 or 3 position by halogen, nitro, methyl, methoxy, difluoromethoxy, trifluoromethoxy, dimethyl or diethylamino, methylthio or trifluoromethyl, or a disubstituted phenyl residue substituted, preferably in the 2,3 position, by methoxy or methylenedioxy, or in the 2,3 or 2,6 position by halogen atoms, which may be the same or different,

$R^2$ represents a nitrile group, a carboxyl group or an alkoxycarbonyl residue of the general formula II

$$-CO_2R^5 \qquad (II)$$

in which $R^5$ represents a methyl, ethyl, n-propyl, isopropyl, n-butyl, isobutyl, tert. butyl or a lower alkoxyalkyl residue,

$R^3$ represents a methyl or ethyl residue or an amino group,

$R^4$ represents hydrogen, a methyl, ethyl, n-propyl or isopropyl residue, an alkoxyalkyl group of the general formula III

$$-(CH_2)_n-O-R^6 \qquad (III)$$

in which $R^6$ represents a lower straight-chain or branched alkyl group and n has the value 2 or 3, or an aminoalkyl group of the general formula IV

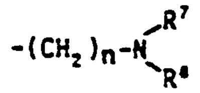

(IV)

in which $R^7$ and $R^8$ may be the same or different, and represent a straight-chain or branched lower alkyl group, or together form a lower alkylene group, and n is 2 or 3, as well as, optionally, pharmacologically harmless salts thereof.

2. Process for the preparation of 4-oxo-pyrido [2,3-d] pyrimidine derivatives according to claim 1, characterised in that either

a) a dihydropyridine of the general formula V

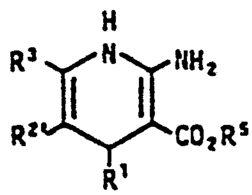

(V)

in which $R^1$ $R^3$ and $R^5$ have the meaning above, and $R^{2'}$ represents a nitrile group or an alkoxycarbonyl residue of the general formula II, is condensed with s-triazine, and the compounds of the general formula VI

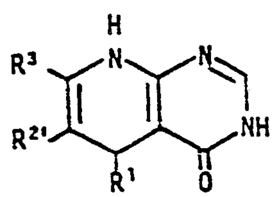

(VI)

thus obtained, in which $R^1$, $R^{2'}$ and $R^3$ have the meaning above, are reacted, optionally and in a known manner, with a compound of the general formula VII

$$X-R^4 \qquad (VII)$$

in which $R^4$ has the meaning above with the exception of hydrogen, and X represents halogen, in particular chlorine, bromine or iodine, or

b) a compound of the general formula VIII

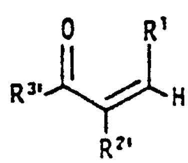

(VIII)

in which $R^1$ and $R^{2'}$ have the above meaning and $R^{3'}$ represents a straight-chain or branched alkyl group of up to 4 carbon atoms, is condensed with 6-amino-4-hydroxy-pyrimidine, and the compounds of the general formula VI thus obtained are reacted with a compound of the general formula VII, if desired.

3. Use of a compound of the general formula I according to claim 1, for the preparation of a medicament for combatting vascular diseases.

4. A medicament containing at least one compound according to claim 1.

**Claims** (for the contracting state AT)

1. Process for the preparation of 4-oxo-pyrido [2,3-d] pyrimidine derivatives of the general formula I

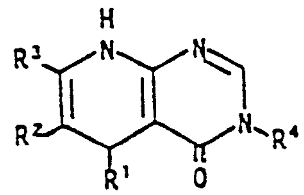

(I)

in which

$R^1$ represents an unsubstituted phenyl residue or a substituted phenyl residue, substituted in the 2 or 3 position by halogen, nitro, methyl, methoxy, difluoromethoxy, trifluoromethoxy, dimethyl or diethylamino, methylthio or trifluoromethyl, or a disubstituted phenyl residue substituted, preferably in the 2,3 position, by methoxy or methylenedioxy, or in the 2,3 or 2,6 position by halogen atoms, which may be the same or different,

$R^2$ represents a nitrile group, a carboxyl group or an alkoxycarbonyl residue of the general formula II

$$—CO_2R^5 \qquad (II)$$

in which $R^5$ represents a methyl, ethyl, n-propyl, isopropyl, n-butyl, isobutyl, tert. butyl or a lower alkoxyalkyl residue,

$R^3$ represents a methyl or ethyl residue or an amino group,

$R^4$ represents hydrogen, a methyl, ethyl, n-propyl or isopropyl residue, an alkoxyalkyl group of the general formula III

$$—(CH_2)_n—O—R^6 \qquad (III)$$

in which $R^6$ represents a lower straight-chain or branched alkyl group and n has the value 2 or 3, or an aminoalkyl group of the general formula IV

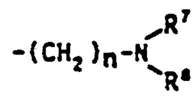

(IV)

in which $R^7$ and $R^8$ may be the same or different, and represent a straight-chain or branched lower alkyl group, or together form a lower alkylene group, and n is 2 or 3, as well as, optionally, pharmacologically harmless salts thereof, characterised in that

a) a dihydropyridine of the general formula V

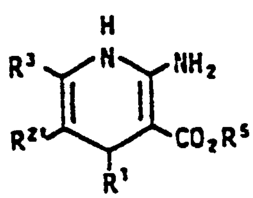

(V)

in which $R^1$, $R^3$ and $R^5$ have the meaning above, and $R^{2'}$ represents a nitrile group or an alkoxycarbonyl residue of the general formula II, is condensed with s-triazine, and the compounds of the general formula VI

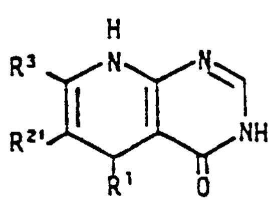

(VI)

thus obtained, in which $R^1$, $R^{2'}$ and $R^3$ have the meaning above, are reacted, optionally and in a known manner, with a compound of the general formula VII

$$X—R^4 \qquad (VII)$$

in which $R^4$ has the meaning above with the exception of hydrogen, and X represents halogen, in particular chlorine, bromine or iodine, or

b) a compound of the general formula VIII

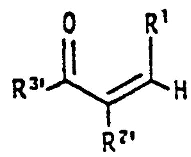

(VIII)

in which $R^1$ and $R^{2'}$ have the meaning above and $R^{3'}$ represents a straight-chain or branched alkyl group of up to 4 carbon atoms, is condensed with 6-amino-4-hydroxy-pyrimidine, and the compounds of the general formula VI thus obtained are reacted with a compound of the general formula VII, if desired, and the compounds I thus obtained are subsequently and optionally transformed into pharmacologically acceptable salts thereof.

2. Use of compounds of the general formula I according to claim 1 for the preparation of a medicament for combatting vascular diseases.

**Revendications** (pour les Etats contractants : BE, CH, DE, FR, GB, IT, LI, LU, NL, SE)

1. 4-oxo-pyrido (2,3-d) pyrimidine de formule générale I :

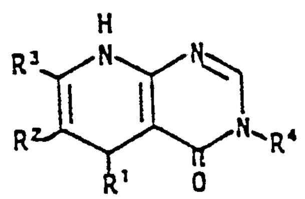

(I)

dans laquelle

$R^1$ représente un radical phényle non substitué ou un radical phényle substitué en position-2 ou en position-3 par un atome d'halogène, un groupe nitro, méthyle, méthoxy, difluorométhoxy, trifluorométhoxy, diméthyle ou diéthylamino, méthylthio ou trifluorométhyle ou un radical phényle disubstitué, de préférence en position-2,3 par des radicaux méthoxy ou méthylènedioxy ou en position-2,3 ou en position-2,6 par des atomes d'halogène qui peuvent être identiques ou différents ;

$R^2$ représente un groupe nitrile, un groupe carboxyle ou un radical alkoxycarbonyle de formule générale II :

$$—CO_2R^5 \qquad (II)$$

dans laquelle

$R^5$ représente un groupe méthyle, éthyle, n-propyle, isopropyle, n-butyle, isobutyle, tertiobutyle, ou un radical alkylalkoxy inférieur ;

$R^3$ signifie un radical méthyle ou éthyle ou un groupe amino ;

$R^4$ signifie un atome d'hydrogène, un radical méthyle, éthyle, n-propyle ou isopropyle, un groupe alkoxyalkyle de formule générale III :

$$—CH(_2)_n—O—R^6 \qquad (III)$$

dans laquelle

$R^6$ représente un groupe alkyle inférieur à chaîne droite ou ramifiée et ;

n est égal à 2 ou à 3, ou un groupe aminoalkyle de formule générale IV :

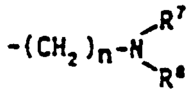

(IV)

dans laquelle

$R^7$ et $R^8$ peuvent être identiques ou différents et représentent un groupe alkyle inférieur à chaîne droite ou ramifiée ou ils forment, ensemble un groupe alkylène inférieur et ;

n est égal à 2 ou 3,

ainsi qu'éventuellement leurs sels pharmaceutiquement compatibles.

2. Procédé de préparation de dérivés de 4-oxo-pyrido(2,3-d) pyrimidine selon la revendication 1, caractérisé en ce que, ou bien :

(a) on condense avec la s-triazine une dihydropyridine de formule générale V :

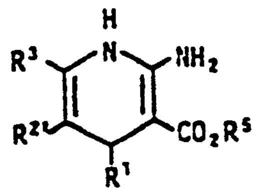

(V)

dans laquelle

$R^1$, $R^3$ et $R^5$ présentent la signification indiquée ci-dessus ; et

$R^{2'}$ représente un groupe nitrile ou un radical alkoxy de formule générale II, et l'on fait réagir les dérivés ainsi obtenus de formule générale VI :

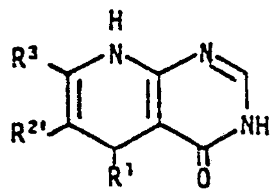

(VI)

dans laquelle

$R^1$, $R^{2'}$ et $R^3$ présentent la signification indiquée ci-dessus, le cas échéant, d'une façon connue en soi, avec un dérivé de formule générale VII :

$$X—R^4 \qquad (VII)$$

dans laquelle

$R^4$ représente la signification indiquée ci-dessus à l'exception de l'hydrogène ; et

X représente un atome d'halogène, notamment chlore, brome ou iode ;

ou bien

(b) on condense avec la 6-amino-4-hydroxypyrimidine, un dérivé de formule générale VIII :

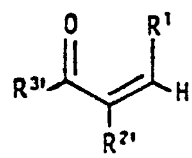

(VIII)

dans laquelle

$R^1$ et $R^{2'}$ présentent la signification indiquée ci-dessus ; et

$R^{3'}$ signifie un radical alkyle à chaîne droite ou ramifiée renfermant jusqu'à 4 atomes de carbone ;

et l'on fait réagir les dérivés de formule générale VI ainsi obtenus éventuellement avec un dérivé de formule générale VII.

3. Utilisation des dérivés de formule générale I selon la revendication 1, à la préparation de médicaments destinés à la lutte contre les maladies des vaisseaux.

4. Médicaments contenant au moins un dérivé selon la revendication 1.

**Revendications** (pour l'Etat contractant AT)

1. Procédé de préparation de 4-oxo-pyrido (2,3-d) pyrimidine de formule générale I :

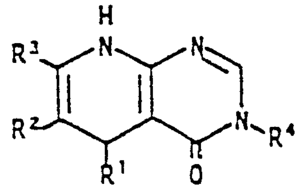

(I)

dans laquelle

$R^1$ représente un radical phényle non substitué ou un radical phényle substitué en position-2 ou en position-3 par un atome d'halogène, un groupe nitro, méthyle, méthoxy, difluorométhoxy, trifluorométhoxy, diméthyle ou diéthylamino, méthylthio ou trifluorométhyle ou un radical phényle disubstitué, de préférence en position-2,3 par des radicaux méthoxy ou méthylènedioxy ou en position-2,3 ou en position-2,6 par des atomes d'halogène qui peuvent être identiques ou différents ;

$R^2$ représente un groupe nitrile, un groupe carboxyle ou un radical alkoxycarbonyle de formule générale II :

$$—CO_2R^5 \qquad (II)$$

dans laquelle

$R^5$ représente un groupe méthyle, éthyle, n-propyle, isopropyle, n-butyle, isobutyle, tertiobutyle, ou un radical alkylalkoxy inférieur ;

$R^3$ signifie un radical méthyle ou éthyle ou un groupe amino ;

$R^4$ signifie un atome d'hydrogène, un radical méthyle, éthyle, n-propyle ou isopropyle, un groupe alkoxyalkyle de formule générale III :

$$—CH(_2)_n—O—R^6 \qquad (III)$$

dans laquelle

$R^6$ représente un groupe alkyle inférieur à chaîne droite ou ramifiée et ;

n est égal à 2 ou à 3, ou un groupe aminoalkyle de formule générale IV :

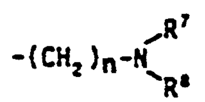

(IV)

dans laquelle

$R^7$ et $R^8$ peuvent être identiques ou différents et représentent un groupe alkyle inférieur à chaîne droite ou ramifiée ou ils forment, ensemble un groupe alkylène inférieur et ;

n est égal à 2 ou 3,

ainsi qu'éventuellement leurs sels pharmaceutiquement compatibles, caractérisé en ce que :

(a) on condense avec la s-triazine une dihydropyridine de formule générale V :

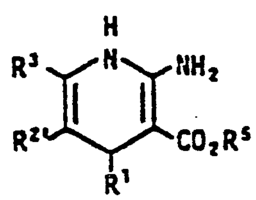

(V)

dans laquelle

$R^1$, $R^3$ et $R^5$ présentent la signification indiquée ci-dessus ; et

$R^{2'}$ représente un groupe nitrile ou un reste alkoxy carbonyle de formule générale II ;

et l'on fait réagir les dérivés ainsi obtenus de formule générale VI :

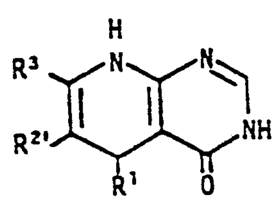

(VI)

dans laquelle $R^1$, $R^{2'}$ et $R^3$ présentent la signification indiquée ci-dessus, le cas échéant, d'une façon connue en soi, avec un dérivé de formule générale VII :

$$X—R^4 \qquad (VII)$$

dans laquelle

$R^4$ représente la signification indiquée ci-dessus à l'exception de l'hydrogène ; et

X représente un atome d'halogène, notamment chlore, brome ou iode ;
ou bien
(b) on condense avec la 6-amino-4-hydroxypyrimidine, un dérivé de formule générale VIII :

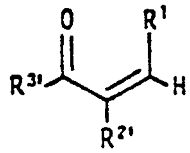

(VIII)

dans laquelle
$R^1$ et $R^{2'}$ présentent la signification indiquée ci-dessus ; et
$R^{3'}$ signifie un radical alkyle à chaîne droite ou ramifiée renfermant jusqu'à 4 atomes de carbone ;
et l'on fait réagir les dérivés de formule générale VI ainsi obtenus éventuellement avec un dérivé de formule générale VII et l'on transforme les dérivés de formule générale I ainsi obtenus en sels pharmaceutiquement compatibles.

2. Utilisation des dérivés de formule générale I selon la revendication 1, à la préparation de médicaments destinés à la lutte contre les maladies des vaisseaux.